# EUROPEAN PATENT APPLICATION

(11) **EP 4 265 718 A1**
(43) Date of publication of application: **25.10.2023**
(21) Application number: 22169518.2
(22) Date of filing: 22.04.2022
(51) Int. Cl.: C12N 9/12

(54) **NOVEL SINGLE-SUBUNIT RNA POLYMERASE**

(71) Applicant: Universität Hamburg, 20148 Hamburg (DE)
(72) Inventor: Streit, Wolfgang, 24248 Moenkeberg (DE); Han, Yuchen, 22607 Hamburg (DE); Kinfu, Birhanu, 24103 Kiel (DE)
(74) Representative: Stüven, Ralf

(57) **Abstract**

The invention relates to a novel DNA-directed single-subunit RNA polymerase. The invention provides a single-subunit RNA polymerase, ssRNAP, the ssRNAP having advantageous features compared to the well-known T7 RNAP.

## Description

The invention relates to a novel DNA-directed single-subunit RNA polymerase.

RNA polymerases, in particular viral DNA-directed single-subunit RNA polymerases (ssRNAP), are frequently used in biotechnology for numerous purposes, for example for overexpression of recombinant genes in vivo or the synthesis of RNA in vitro. The in vitro transcription (IVT) of mRNA, for example, has recently gained importance because of its application in the production of mRNA vaccines (see, e.g., Jeeva, S., Kim, K. H., Shin, C. H., Wang, B. Z., & Kang, S. M. (2021). An Update on mRNA-Based Viral Vaccines. Vaccines, 9(9), 965, doi:10.3390/vaccines9090965; Webb C, Ip S, Bathula NV, Popova P, Soriano SKV, Ly HH, Eryilmaz B, Nguyen Huu VA, Broadhead R, Rabel M, Villamagna I, Abraham S, Raeesi V, Thomas A, Clarke S, Ramsay EC, Perrie Y, Blakney AK, (2022), Current Status and Future Perspectives on mRNA Drug Manufacturing, Mol Pharm. 2022, doi: 10.1021/acs.molpharmaceut.2c00010).

Single-subunit RNA polymerases (ssRNAPs) of viral origin, for example, RNA polymerases of the bacteriophages T7, T3 or SP6, are widely used in biotechnological applications for the synthesis of RNA from DNA templates, because of their structural simplicity, promoter specificity, transcription efficiency and accuracy, and their tolerance for non-canonical ribonucleoside triphosphates (rNTP) like pseudouridine or N1-methylpseudouridine, which is important for the production of mRNA therapeutics (see, for example, Nance K.D. & Meier J.L., 2021, Modifications in an Emergency: The Role of N1-Methylpseudouridine in COVID-19 Vaccines, ACS Cent. Sci. 2021, 7, 5, 748-756, doi.org/10.1021/acscentsci.1c00197). They can be efficiently cloned and expressed (see, e.g., US 4952496). Mutant enzymes with desired properties, for example a different promoter specificity or an mRNA capping activity, have been designed (US 5385834, US 11236311 B2).

There is still a need, however, for alternative RNA polymerases for use in biotechnological applications.

In a first aspect the invention provides a single-subunit RNA polymerase, ssRNAP, the ssRNAP comprising
a) an amino acid sequence according to the sequence of SEQ ID NO: 1, or
b) an amino acid sequence having at least 75% sequence identity with the amino acid sequence of SEQ ID NO: 1.

The single-subunit RNA polymerase according to the invention, also designated RNAP_E in the following, is highly efficient and can be stably stored under suitable conditions over a long period of time. It has its maximum activity at 37 °C, but compared to the activity at 37°C after one hour, the RNAP of the invention still exhibits around 52 % activity at 45°C and 55°C and 29-31 % activity at 65°C and 75°C, and is thus more thermostable than the known T7 RNA polymerase. Further, the RNAP of the invention was, at least in the first 25 minutes, 3.2 faster at 37 °C than T7 RNAP, and has a comparatively high salt tolerance. The RNA polymerase according to the invention has only 26% identity (calculated by NCBI BLAST Global Alignment, using standard parameters) with T7 RNA polymerase based on amino acid alignment. The highest identity (71%) observed was with a DNA-directed RNA polymerase from *Enterobacter cloacae* phage phiKDA1 (YP_009167685.1, GeneId: 26040818).

The RNA polymerase of the invention can be used in numerous biotechnological applications, for example for the biotechnological production of RNA, e.g., mRNA, in particular mRNA therapeutics like, inter alia, mRNA vaccines, the amplification of RNA in metagenomic screening methods, the generation of DNA/RNA hybrids, analytical methods, diagnostic methods, structural studies, probe generation and several other biotechnological applications.

The term "single-subunit RNA polymerase", abbreviated ssRNAP or RNAP, refers to a DNA-directed (DNA-dependent) RNA polymerase, i.e., a DNA-directed nucleoside-triphosphate:RNA nucleotidyltransferase, catalyzing the DNA-template-directed extension of the 3'-end of an RNA strand by one nucleotide at a time (EC 2.7.7.6). The term "single-subunit" refers to the fact that the RNAP is composed of a single peptide molecule. The terms "peptide" and "protein" are used synonymously herein.

The term "comprising" as used herein encompasses the term "having", i.e., is not to be construed as meaning that further elements have necessarily to be present in an embodiment in addition to the element explicitly mentioned. For example, the term "ssRNAP comprising an amino acid sequence according to SEQ ID NO:X" also encompasses an enzyme having the amino acid sequence according to SEQ ID NO:X, "having" in this context meaning being only composed of the amino acids in SEQ ID NO:X.

The term "% identity" in relation to an amino acid or a nucleotide sequence refers to the percentage of identical amino acids or nucleotides in a given peptide or nucleic acid, compared to a reference peptide or nucleic acid. Identity means that, when comparing two sequences at equivalent positions, i.e., after alignment of the sequences, the same nucleotide or amino acid is present. It may optionally be necessary to take sequence gaps into account in order to produce the best possible alignment. Comparison of two sequences as regards the percentage of identity can, for example, be performed using NCBI BLAST ("Basic Local Alignment Search Tool", https://blast.ncbi.nlm.nih.gov/Blast.cgi). A "global alignment" aligns two sequences from beginning to end, aligning each letter in each sequence only once. A global alignment algorithm may synonymously be referred to as a Needleman-Wunsch algorithm (Needleman, S.B., Wunsch, C.D. (1970), A general method applicable to the search for similarities in the amino acid sequence of two proteins, Journal of Molecular Biology 48 (3): 443-53, doi:10.1016/0022-2836(70)90057-4; Stephen F. Altschul, Thomas L. Madden, Alejandro A. Schäffer, Jinghui Zhang, Zheng Zhang, Webb Miller, and David J. Lipman (1997), Gapped BLAST and PSI-BLAST: a new generation of protein database search programs, Nucleic Acids Res. 25:3389-3402; Stephen F. Altschul, John C. Wootton, E. Michael Gertz, Richa Agarwala, Aleksandr Morgulis, Alejandro A. Schäffer, and Yi-Kuo Yu (2005), Protein database searches using compositionally adjusted substitution matrices, FEBS J. 272:5101-5109). The skilled person, using his expert knowledge, will readily determine which of the available BLAST programs, e.g., BLASTp, PLASTn, Needleman-Wunsch Global Align Protein Sequences, or Needleman-Wunsch Global Align Nucleotide Sequences, is suitable for determination of sequence identity. Unless expressly stated otherwise, or unless the context clearly indicates otherwise, the standard parameters of any of the programs are used. Standard parameters of the Needleman-Wunsch global alignment tool for proteins are: Gapcosts 11,1, BLOSUM62 matrix, of the Needleman-Wunsch global alignment tool for nucleic acids: Match/Mismatch scores 1,-2, Gapcosts 5,2. In addition, the skilled person is aware of further programs for assessing homology, which may be used, if necessary. Such programs are, for example, available on the website of the European Bioinformatics Institute (EMBL) (see, e.g., https://www.ebi.ac.uk/services). Where such terms like "x % homologous to" or "homology of x %" are used herein, this is to be construed as meaning that two proteins or nucleic acids are considered homologous and have a sequence identity of x %, e.g., 80 %. Unless expressly stated otherwise, or unless the context clearly indicates otherwise, a sequence identity of x %, e.g., 85 %, means a percentage of sequence identity determined with the appropriate BLAST global alignment tool mentioned above using standard parameters.

For reasons of clarity it is to be noted that the term "ssRNAP comprising an amino acid sequence having at least 75% sequence identity with the amino acid sequence of SEQ ID NO: 1" implies that the ssRNAP has RNA polymerase activity.

The term "fragment" in relation to a protein or nucleic acid refers to a part of the protein or the nucleic acid, e.g., 100 consecutive amino acids of a protein having 800 amino acids as a whole. The term "fragment having RNA polymerase activity" means that the isolated fragment has RNA polymerase activity under suitable conditions, in particular that the isolated fragment has a measurable RNA polymerase activity under conditions under which a full-length RNA polymerase, for example the full-length RNA polymerase of the invention having the amino acid sequence of SEQ ID NO: 1, has RNA polymerase activity, preferably optimum activity. For example, a fragment is considered to have RNA polymerase activity if the fragment has at least an RNA polymerase activity of 3 %, 4 % or 5 %, preferably at least 10 %, 20 %, 25 % or 30 %, more preferably at least 35 %, 40 %, 45 %, 50 %, 55 % or 60 % of a full length RNA polymerase, e.g., the full-length RNA polymerase of the invention having the amino acid sequence of SEQ ID NO: 1. The activity is preferably compared under optimum conditions for the full-length RNA polymerase, e.g. at a temperature of 37 °C and an optimum pH and salt concentration, for example. It is to be noted here for clarity that a fragment is also considered to have RNA polymerase activity, if the fragment shows RNA polymerase activity under the conditions chosen, where the whole-length RNA polymerase has less activity than the fragment or no activity under these conditions.

The term "transcription regulatory element" relates to any element of a transcription unit regulating the quantity and/or rate of transcription of the transcription unit. Examples of such elements are promoters and enhancers, i.e., segments of DNA containing sequences capable of providing promoter and enhancer functions.

The term "promoter" relates to a sequence of DNA to which proteins bind that initiate transcription of a DNA sequence into an RNA. Usually, a promoter is arranged towards the 5' end of the sense strand of the DNA.

The term "transcription unit" relates to a sequence of nucleotides in DNA that is transcribed into a single RNA molecule, e.g., pre-mRNA or mRNA molecule. The term encompasses the term "gene" referring to a sequence of nucleotides in DNA encoding a peptide.

The term "functionally linked to the promoter" means that the transcription unit is transcribed under the control of the promoter.

The term "nucleotide sequence having promoter activity in relation to the ssRNAP" means that, the nucleotide sequence functions as a promoter for the ssRNAP of the invention, such that a transcription unit is transcribed by the ssRNAP when functionally linked to the promoter. This includes that any nucleotide sequence functioning as a promoter for any one of the ssRNAPs as defined herein in feature b) above and having a first amino acid sequence is considered to have "promoter activity in relation to the ssRNAP of the invention", even if the nucleotide sequence does not or only less efficiently function as a promotor for another ssRNAP as defined in feature b) above and having a second amino acid sequence differing from the first amino acid sequence. It is, however, preferred, that any of the promoter sequences defined herein has promoter activity in relation to any ssRNAP as defined herein.

The terms "non-canonical NTP" or "non-standard NTP" relate to a nucleoside triphosphate, preferably a ribonucleoside triphosphate (rNTP), that is not a "standard" nucleoside triphosphate, "standard" nucleoside triphosphates being thymidine triphosphate, uridine triphosphate, adenosine triphosphate, guanosine triphosphate and cytidine triphosphate. "Standard" ribonucleoside triphosphates (rNTPs) are uridine triphosphate, adenosine triphosphate, guanosine triphosphate and cytidine triphosphate. Examples of a non-canonical NTP are pseudouridine (Ψ) or N1-methylpseudouridine (m1Ψ).

The term "cell" or "host cell" as used herein relates to a living biological, e.g. prokaryotic or eukaryotic cell. Non-limiting examples of eukaryotic cells include plant cells, fungal cells, mammalian cell, non-human animal cells or human cells, preferably with the proviso that the cell is not a human germ cell. The eukaryotic cell may, for example, be a blood cell, epithelial cell or a stem cell (e.g. embryonic stem cells, induced pluripotent stem cell, hematopoietic stem cell). The cell may be a mammalian cell of the orders rodenta (mice, rats, hamsters), lagomorpha (rabbits), carnivora (cats, dogs), and artiodactyla (cows, pigs, sheep, goats, horses). The cell may be from any organism, for example plant, non-human animal, human, non-human primate, mouse, rat, rabbit, cat or dog, preferably with the proviso that it is not a human germ cell. The cell may be isolated or may be part of an organism (e.g., subject), but preferably with the proviso that it is not a human germ cell. Procaryotic cells include, for example, bacterial cells, e.g., *Escherichia coli* cells, and archaean cells.

The term "biotechnological applications" relates to applications in which living cells or functional parts or components of cells, e.g., biomolecules like nucleic acids or proteins, are employed for a specific purpose, for example a therapeutic, diagnostic, analytical, screening or industrial purpose. The term encompasses, for example, the in-vitro synthesis, manufacture, amplification, repair, fusion, assembly or alteration of nucleic acids or other biomolecules, e.g., any DNA or RNA species like mRNA, tRNA, rRNA, small non-coding RNA, e.g., miRNA or siRNA. The term also includes any purposeful use of a cell or a cellular component or biomolecule, e.g., a protein or nucleic acid, in an analytical, diagnostic, therapeutic or other medical method, for example, in prognosis, prevention or palliation of any disorder of a structure or function in a human, animal, or plant cell. The in-vitro production of RNA therapeutics like, inter alia, mRNA vaccines or RNA gene therapeutics is an example of a biotechnological application of a biotechnological application of the RNAP of the invention.

In a preferred embodiment, the ssRNAP of the invention has at least 80 %, preferably at least 85 %, 90%, 95%, 96%, 97%, 98%, 99%, or at least 99.5% sequence identity with the amino acid sequence of SEQ ID NO: 1.

It is particularly preferred that an ssRNAP of the invention having at least 75 %, at least 80 %, 85 %, 90%, 95%, 96%, 97%, 98%, 99%, or at least 99.5% sequence identity with the amino acid sequence of SEQ ID NO: 1 has a higher temperature tolerance, higher tolerance to non-canonical NTPs, a different promotor specifity, higher transcription activity, or higher transcription accuracy, compared to the ssRNAP of the invention having an amino acid sequence of SEQ ID NO:1. Methods for making and isolating suitable enzyme variants are known to the skilled person, and include different in vitro evolution techniques (see, for example, Golynskiy MV, Haugner JC 3rd, Morelli A, Morrone D, Seelig B. In vitro evolution of enzymes. Methods Mol Biol. 2013;978:73-92. doi:10.1007/978-1-62703-293-3_6).

In a second aspect the invention also relates to a fragment of an ssRNAP according to the first aspect, wherein the fragment comprises at least 60, preferably at least 65, 70, 75, 80, 85, 90, 100, 110 or at least 120 consecutive amino acids of said enzyme, and wherein the fragment has RNA polymerase activity. Further preferred, the ssRNAP fragment comprises at least 150, 175, 200, 250, 300, 350, 400, 450, 500, 550, 600, 650, 700 or at least 750 consecutive amino acids of the ssRNAP, and wherein the fragment has RNA polymerase activity.

In a third aspect the invention relates to a fusion protein (chimeric protein), comprising an ssRNAP according to the first aspect of the invention, or a fragment of an ssRNAP according to the second aspect of the invention, fused to a protein being different from the ssRNAP of the first aspect of the invention, or from a fragment according to the second aspect of the invention, preferably a protein having an enzymatic activity different from an RNA polymerase. The fusion protein may comprise more than one protein or protein domain fused to the ssRNAP of the invention or a fragment thereof as defined above. The protein fused to the ssRNAP may, for example, be a capping enzyme or a peptide domain having capping activity. A capping enzyme is an enzyme adding a 5'-cap, e.g. a 7-methylguanosine (m7G) cap, to an mRNA. Preferably, the capping enzyme is a single-subunit enzyme like the capping enzyme NP868R from African swin fever virus (ASFV, see, for example, Eaton, H. E., Kobayashi, T., Dermody, T. S., Johnston, R. N., Jais, P. H., & Shmulevitz, M. (2017). African Swine Fever Virus NP868R Capping Enzyme Promotes Reovirus Rescue during Reverse Genetics by Promoting Reovirus Protein Expression, Virion Assembly, and RNA Incorporation into Infectious Virions. Journal of virology, 91(11), e02416-16. https://doi.org/10.1128/JVI.02416-16). The protein may be fused directly or via a linker peptide to the N-terminus or the C-terminus of the ssRNAP of the invention, or a fragment thereof according to the second aspect of the invention. The fusion protein may be used for different purposes, for example, for producing an mRNA capped with a 5'-cap.

The ssRNAP of the first aspect of the invention, the fragment of the second aspect of the invention, or the fusion protein of the third aspect of the invention preferably interact, more preferably specifically interact, with a promotor described below in relation to a fifth aspect of the invention. The term "interact with a promoter" means in this context that the ssRNAP of the first aspect of the invention, the fragment of the second aspect of the invention, or the fusion protein of the third aspect of the invention transcribe a given target nucleic acid functionally linked to the promoter under the control of the promoter.

In a fourth aspect the invention relates to a nucleic acid, preferably a DNA, being or comprising a nucleic acid encoding an ssRNAP according to the first aspect of the invention, or a fragment according to the second aspect of the invention or a fusion protein according to the third aspect of the invention.

The nucleic acid may be a linear or circular, single- or double-stranded nucleic acid, preferably DNA, for example a vector, such as a plasmid, viral vector, cosmid or artificial chromosome. The nucleic acid can, for example, serve as a template for in vitro transcription of messenger RNA (mRNA), or as a means to introduce the nucleic acid into a host cell, e.g., a fungal, bacterial or plant cell. The nucleic acid may be functionally linked to a suitable promoter and/or other transcription regulatory elements in order to achieve expression of the nucleic acid in a host cell. The host cell can be a procaryotic or eucaryotic cell, for example, a bacterial cell, a plant cell, a fungal cell, e.g. yeast cell, or an animal cell, preferably non-human animal cell. The nucleic acid of this fourth aspect can, for example, be used as a storage molecule for the ssRNAP, fragment or fusion protein of the invention, or for the production of the ssRNAP, fragment or fusion protein of the invention.

In a fifth aspect the invention relates to a nucleic acid, preferably a DNA, comprising a promoter and a transcription unit functionally linked to the promoter, the promoter being or comprising
a) the nucleotide sequence of SEQ ID NO: 2 (5'-TCAGAAGTCACACTATAA-3') or SEQ ID NO: 3 (5'-AATAGAAACATACTATAA-3'), preferably SEQ ID NO: 2, or
b) a nucleotide sequence having at least 75 % sequence identity with the nucleotide sequence of SEQ ID NO: 2 or 3, preferably SEQ ID NO: 2, the nucleotide sequence having promoter activity in relation to the ssRNAP according to the invention. The promoter nucleotide sequence preferably has a sequence length of 16-20, more preferably of 17-19, most preferred of 18 nucleotides.

The nucleic acid can, for example, be used for the production of a desired target molecule, e.g. an mRNA molecule suitable as a vaccine or other therapeutic, an mRNA molecule suitable for the in vitro production of a protein or an mRNA or other RNA molecule suitable for a diagnostic purpose. The transcription unit to be transcribed into the desired target molecule is transcribed under the control of the promotor as defined in a) or b) above by an ssRNAP, fragment or fusion protein of the invention.

The promoter sequence of SEQ ID NO: 2 is preferred as the promoter sequence of the ssRNAP of the invention, because it produced the highest amount of RNA from a DNA template containing the promoter sequence. The promoter has 67 % identity (calculated by NCBI Blast Global Alignment) with the T7 promoter sequence. The ssRNAP of the invention was moderately active on the promoter of SEQ ID NO: 3, having 68 % identity with the promoter according to SEQ ID NO: 2. The ssRNAP of the invention with SEQ ID NO: 1 binds specifically to this promoter sequence. The transcription unit may, for example, be a gene of interest to be transcribed in vitro into an mRNA by the ssRNAP of the invention. The mRNA may, for example, be an mRNA therapeutic, i.e. be useful as a drug, e.g., as a vaccine or drug used in gene therapy. Examples of mRNA vaccines that may be produced as transcription products are, for example mRNA vaccines against SARS-CoV-2 virus (COVID-19) or other corona viruses, respiratory syncytial virus (RSV), influenza virus, herpes simplex virus (HSV), hepatitis virus, human papillomavirus (HPV), zika virus (ZIKV), rabies virus, HIV, malaria, or tuberculosis. Further examples of mRNA therapeutics are mRNA suitable for prevention or treatment of Alzheimer's disease, asthma, diabetes, HIV, cancer or cardiovascular disease. The RNA produced may also be used for diagnostic or analytic purposes.

In a preferred embodiment, the nucleic acid comprises a promoter, the promoter being or comprising a nucleotide sequence having at least 80 %, preferably at least 85 %, 90%, 95%, 96%, 97%, 98%, 99%, or at least 99.5% sequence identity with the nucleotide sequence of SEQ ID NO: 2 or 3, preferably the nucleotide sequence of SEQ ID NO: 2, the nucleotide sequence having promoter activity in relation to the ssRNAP according to the first aspect of the invention, a fragment according to the second aspect of the invention, and/or the fusion protein according to the third aspect of the invention.

Preferably, any of the promoters described above have promoter activity in relation to the ssRNAP, the fragment and/or the fusion protein of the invention.

In a sixth aspect the invention relates to a vector comprising a nucleic acid according to the fourth or fifth aspect of the invention. A vector is any particle used in biotechnology for the artificial introduction of a foreign nucleic acid into a host cell, for example a bacterial or eukaryotic cell. Suitable vectors and their application are known to the skilled person, and include, for example, plasmids, cosmids, viral vectors and artificial chromosomes. A preferred embodiment of a vector is an expression vector, e.g., a plasmid, comprising a transcription unit and a suitable promoter sequence, the promoter sequence being or comprising
a) the nucleotide sequence of SEQ ID NO: 2 or 3, or
b) a nucleotide sequence having at least 75 %, 80 %, 85 %, 90%, 95%, 96%, 97%, 98%, 99%, or at least 99.5% sequence identity with the nucleotide sequence of SEQ ID NO: 2 or 3, preferably SEQ ID NO: 2, the nucleotide sequence having promoter activity in relation to the ssRNAP according to the first aspect of the invention, or the fragment according to the second aspect of the invention, and/or the fusion protein according to the second aspect of the invention.

In a seventh aspect the invention relates to a host cell being transformed with a nucleic acid according to the fourth or fifth aspect of the invention, or with a vector according to the sixth aspect of the invention. The host cell may be a prokaryotic or eukaryotic host cell, for example an *E. coli* cell, an insect cell, or a mammalian cell. In case of a mamalian cell, the cell is preferably not a human germ cell.

In a further aspect the invention relates to a composition, the composition comprising an ssRNAP according to the first aspect of the invention, a fragment thereof according to the second aspect of the invention, a fusion protein according to the third aspect of the invention, or a nucleic acid according to the fourth or fifth aspect of the invention.

In a further aspect the invention relates to the use of an ssRNAP according to the first aspect of the invention or the use of a fragment according to the second aspect of the invention, or of a fusion protein according to the third aspect of the invention, for the biotechnological production of RNA, for example mRNA, or RNA/DNA hybrid molecules (DNA:RNA hybrid duplexes; heteroduplexes), i.e. double-stranded molecules composed of a DNA strand and a complementary RNA strand. The mRNA may, for example, be an mRNA therapeutic, e.g. an mRNA used in gene therapy or a mRNA vaccine. Examples of mRNA vaccines that may be produced are, for example mRNA vaccines against SARS-CoV-2 virus (COVID-19) or other corona viruses, respiratory syncytial virus (RSV), influenza virus, herpes simplex virus (HSV), hepatitis virus, human papillomavirus (HPV), zika virus (ZIKV), rabies virus, HIV, malaria, or tuberculosis. Further examples of mRNA therapeutics are mRNA suitable for prevention or treatment of Alzheimer's disease, asthma, diabetes, HIV, cancer or cardiovascular disease. The RNA produced may also be used for diagnostic or analytic purposes.

In a further aspect the invention relates to a method of amplifying a target nucleic acid in vitro, comprising the step of contacting the ssRNAP according to the first aspect of the invention, a fragment thereof according to the second aspect of the invention, or a fusion protein according to the third aspect of the invention with a DNA template comprising the target nucleic acid, and with nucleoside triphosphate molecules, NTPs, suitable or necessary for the in vitro transcription of the target nucleic acid, under conditions suitable for a transcription of the target nucleic acid to occur. Suitable conditions for a transcription reaction are well known to the skilled person, and can also be derived from the following examples. Suitable NTPs may also include non-canonical NTPs.

The method of the invention can, for example, be used for the amplification of RNA, for example large-scale production of mRNA, e.g., mRNA therapeutics, or used in metagenomics, i.e., for metagenome mining. The term "amplification of RNA" encompasse the synthesis of RNA/DNA hybrid duplexes. The method can, for example, include the amplification of RNA from a DNA plasmid as a DNA template. Other DNA templates, e.g., linear or linearized DNA molecules, can, however, also be used in the method of the invention. The method can also be used for the in-vitro production of small amounts of RNA or RNA/DNA hybrid duplexes, e.g., for use in assays and test methods.

In a preferred embodiment of the method of the invention, the target nucleic acid is functionally linked to a promoter having the sequence according to SEQ ID NO: 2 or 3, or to a promoter having a nucleotide sequence having at least 75 % sequence identity, preferably at least 80 %, 85 %, 90%, 95%, 96%, 97%, 98%, 99%, or at least 99.5% sequence identity with the nucleotide sequence of SEQ ID NO: 2 or 3, the nucleotide sequence having promoter activity in relation to the ssRNAP according to the invention.

In a further preferred embodiment of the method of the invention, the target nucleic acid is an mRNA therapeutic, for example an mRNA vaccine or an mRNA for use in gene therapy.

In a still further aspect, the invention relates to a kit for carrying out the method of the invention, the kit comprising an ssRNAP according to the first aspect of the invention, or a fragment thereof according to the second aspect of the invention, or a fusion protein according to the third aspect of the invention, and a buffer suitable for the transcription of the target nucleic acid to occur. In a preferred embodiment the kit additionally comprises a nucleic acid according to the fourth aspect of the invention or according to the fifth aspect of the invention, preferably a nucleic acid according to the fifth aspect of the invention. For example, the kit can comprise a nucleic acid comprising a particular transcription unit under the control of a promoter as described above in the context of the nucleic acid according to the fifth aspect of the invention. The kit may also comprise means and/or reagents for replacing the transcription unit for another transcription unit.

The invention will now be described for illustrative purposes only by means of the following figures and examples.

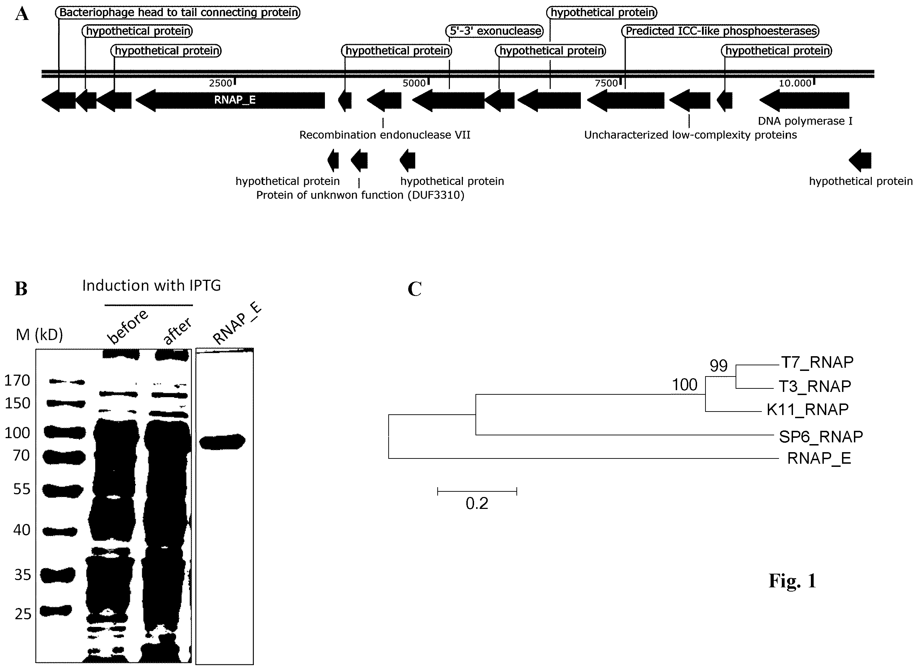

Figure 1. (A) Putative ORFs on the metagenomic DNA. The viral RNA polymerase hit (black marked) was identified on the metagenome obtained from an elephant fecal microbiome in Hamburg Zoo (IMG Submission ID: 16012, Ilmberger et al., 2014) and termed as RNAP_E. The arrows indicate the direction of transcription. (B) Overexpression of RNAP_E. RNAP_E was overexpressed in a vector pET28a(+) with *E*. *coli* BL21(DE3) as host. The cell extracts before and after induction were normalized to OD600. The protein was purified with Ni-NTA agarose, dialyzed with storage buffer and visualized in 10 % SDS-polyacrylamide gel after Coomassie blue staining. (C) The phylogenetic relation of five bacteriophage RNA polymerases. The phylogenetic tree was constructed with MEGA6 (Tamura et al., 2013) based on the Maximum-likelihood method with 1000 bootstrap replications after T-Coffee multiple sequence alignment (Notredame et al., 2000). The percentage of bootstrap resamplings is indicated on the branches. The scale bar represents the expected number of changes per amino acids position.

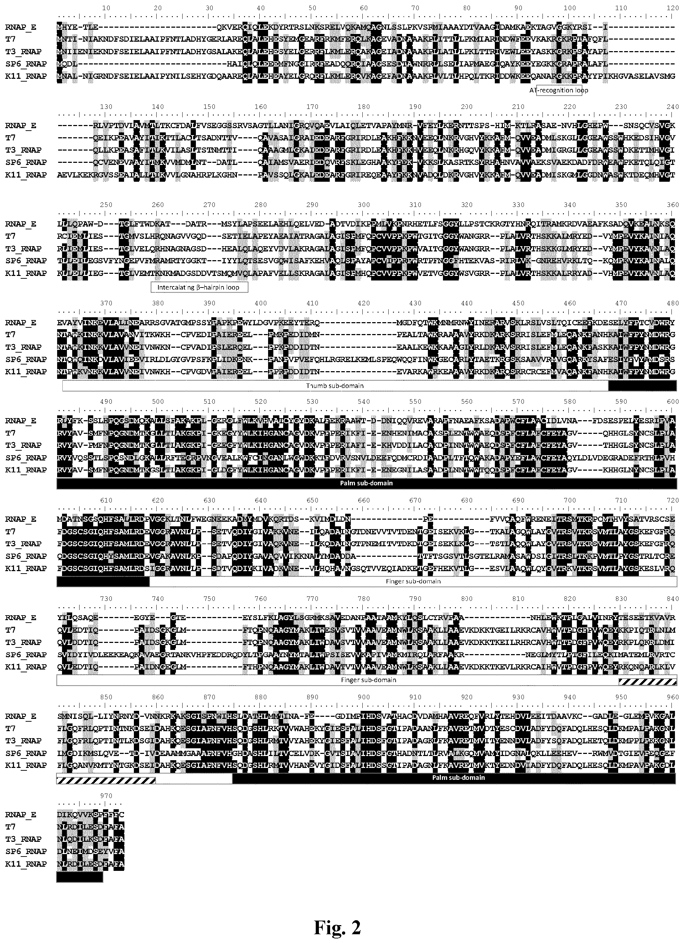

Figure 2. Comparison of RNAP_E protein sequence with other bacteriophage RNA polymerases. The sequence alignment with T-Coffee multiple sequence alignment (Notredame et al., 2000), conserved sites of identity ≥ 80 % are black-shaded and similarity ≥ 80 % are grey-shaded. The color blocks under the sequences indicate the structural and functional domains of RNAP_T7 (Borkotoky and Murali, 2018). RNAP_T7 consists an N-terminal domain and a polymerase domain. The N-terminal domain contains an AT-recognition loop and an intercalating β-hairpin loop, which are important for binding the promoter and placing the template into active site. The polymerase domain contain three sub-structural domains, which consist critical catalytic residues and play a key role for DNA-binding. In the finger sub-domain a specificity loop (box with diagonal line) interacts with promoter.

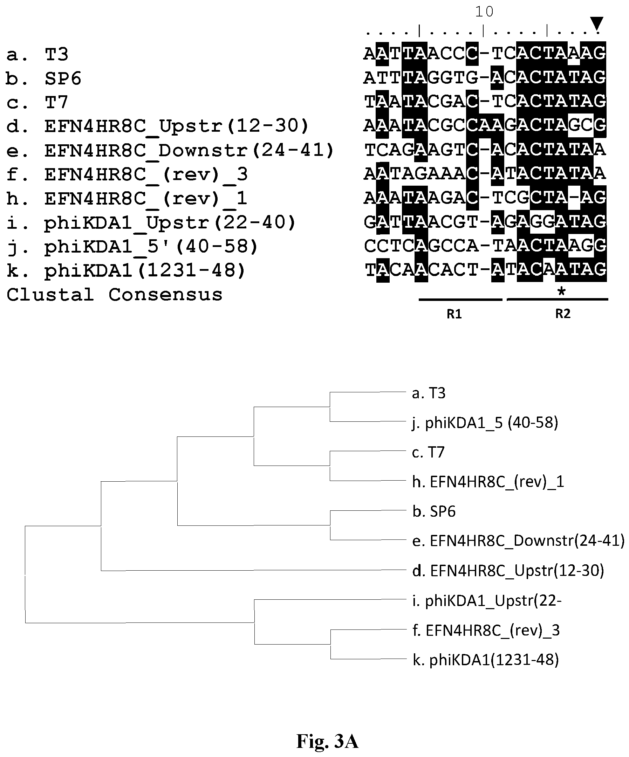

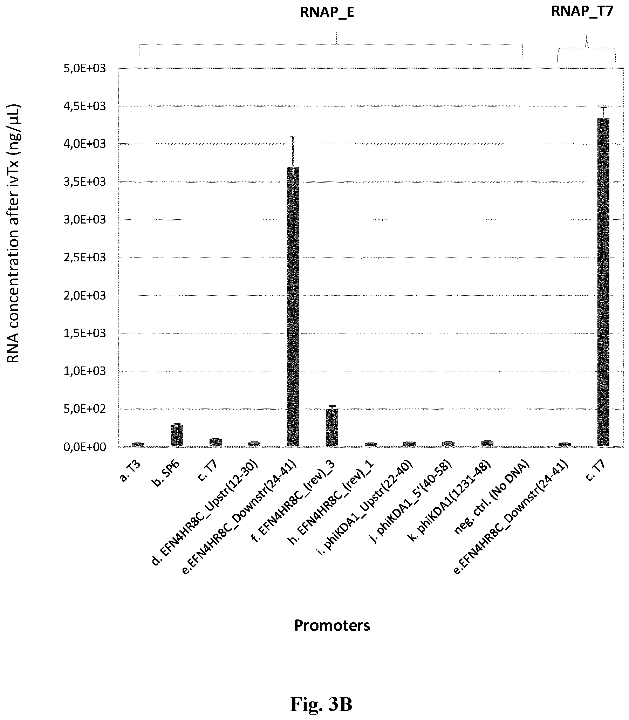

Figure 3. RNAP_E activity with different promoter sequences. (A) Comparison of consensus sequences from candidate promoters. Sequence alignments (upper panel) of candidate sequences and determination of consensus regions were performed to identify potential promoters (Table 2). The candidate promoter sequences were aligned with T-Coffee multiple sequence alignment, conserved sites (identity ≥ 60 %) are black-shaded; asterisk (^{∗}) indicates a single and fully conserved nucleotide; start (+1) is marked by a black triangle; R1 is the -8 to - 12 region, where promoter-specific contacts are being made in this region; R2 is the -7 to +1 region that plays a common function in promoter binding (Jorgensen et al., 1991). The phylogenetic tree of all test promoter sequences (lower panel) was constructed with MEGA-X (Tamura et al., 2013) based on the Maximum-likelihood method with 1000 bootstrap replications after T-Coffee multiple sequence alignment (Notredame et al., 2000). (B) in vitro produced mRNA amount with RNAP_E and RNAP_T7. Candidate promoter sequences were clone-free integrated into PET2 gene fragment using PCR primers carrying them (examples shown underlined, Table 2). Promoter d-h were derived from the metagenome, where the RNAP_E was found; and Promoter i-k were derived from phage phiKDA1. in vitro transcriptions were performed with RNAP_E and RNAP_T7 at 37°C. mRNA concentration was quantified with Nanodrop 2000 spectrophotometer after in vitro transcription (ivTx). Error bars indicate the standard deviation from thee independent experiments.

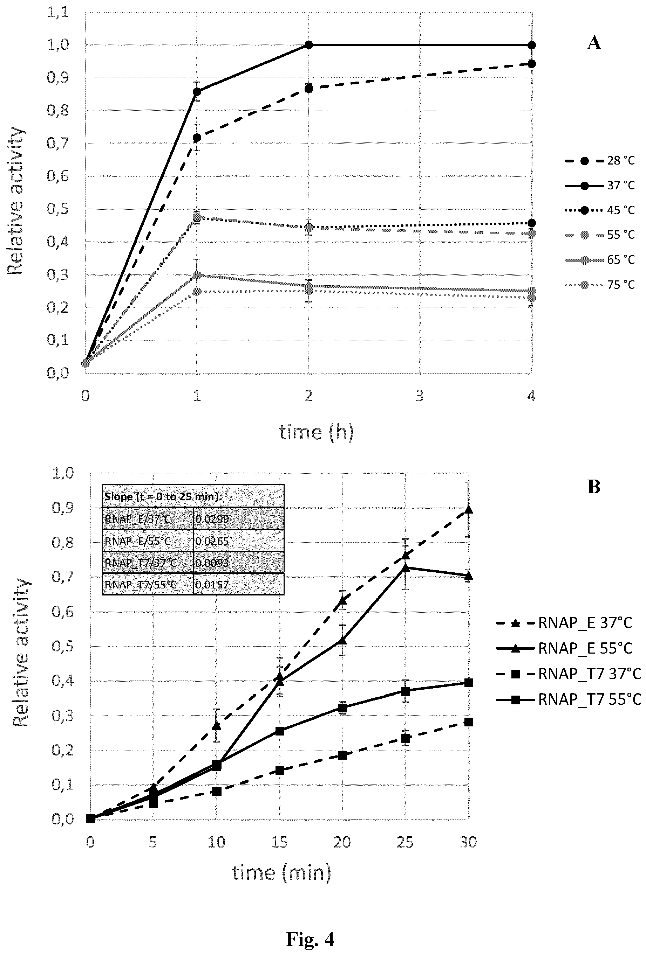

Figure 4. The activity of RNAP_E and RNAP_T7 at different temperature. (A) Relative activity of RNAP_E at 28°C (black dash line), 37°C (black solid line), 45°C (black dot line), 55°C (grey dash line), 65°C (grey solid line), and 75°C (grey dot line). (B) Relative activity of RNAP_E (triangle) and RNAP_T7 (square) at 37°C (dash line) and 55°C (solid line). PET2 DNA sequence was amplified with the primers harboring Promoter e or Promoter c (T7 promoter) and used as template for in vitro transcription with either 5 µg of RNAP_E or 56 unit of RNAP_T7 (Thermo Scientific) at indicated temperature mRNA concentration was quantified with Qubit 3.0 fluorometer using Qubit^{®} RNA HS Assay Kits. And the relative activity is normalized with the amount of in vitro produced RNA at 37°C for 2 h. Error bars indicate the standard deviation from thee independent experiments.

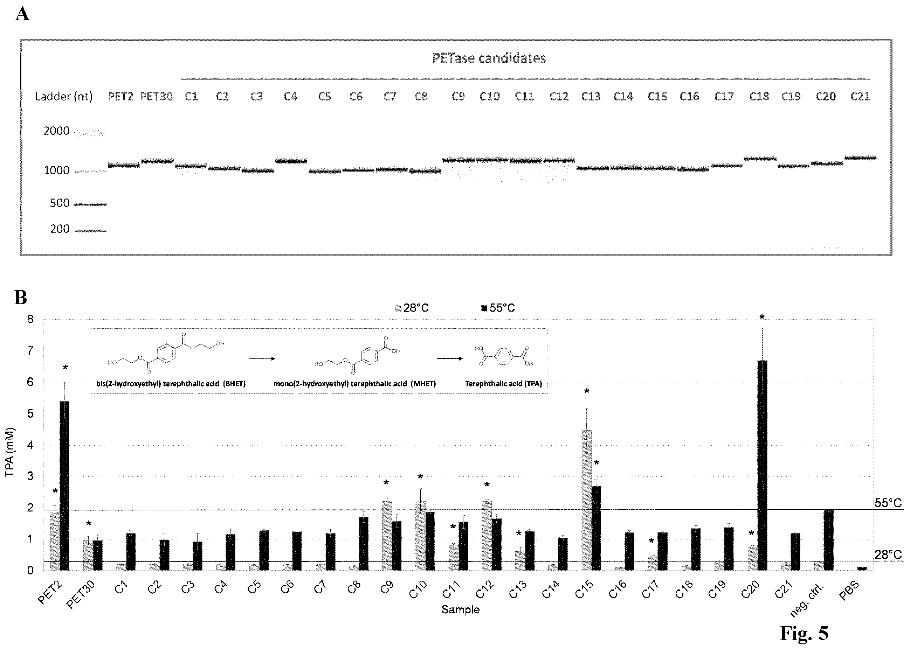

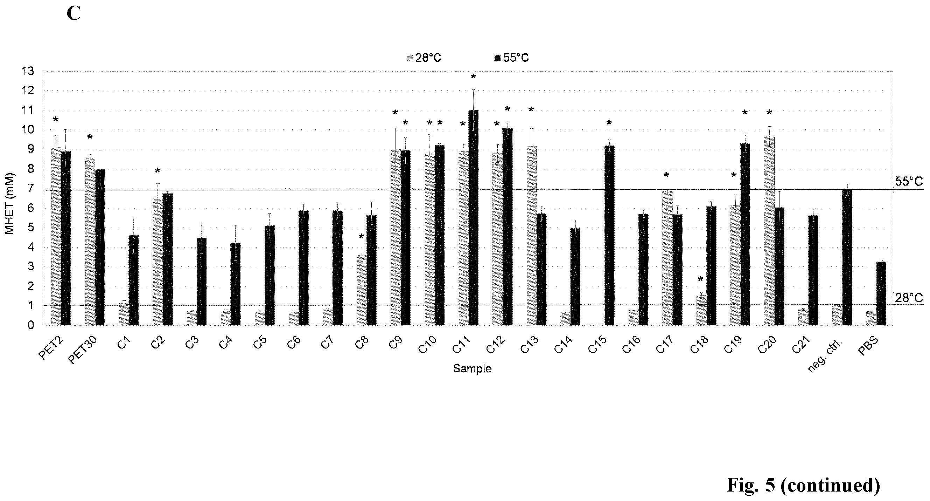

Figure 5. (A) RNA gel *of in vitro* produced mRNAs from PETase candidates with RNAP_E. PETase candidates were identified from different metagenomes (Danso 2018, PG 2021 and Zhang 2021) and their genes were synthesized in a pET vector. Then the DNA sequences were amplified with the primers harboring Promoter e and used as templates for *in vitro* transcription with RNAP_E. RNA quality was controlled with Agilent RNA 6000 Pico chip in Agilent 2100 Bioanalyzer and the RNA gel was derived with 2100 Expert Software. (B, C) Activity *of in vitro* produced PETase candidates 28°C (column with diagonal line) and 55°C (filled column). BHET was used as substrate and the amount of produced TPA (B) and MHET (C) were quantified by UHPLC. The error bars indicate the standard error from three independent experiments and the asterisks (^{∗}) indicate significantly higher amount of TPA than the negative control with the p-value < 0.05. Two well characterized PETases PET2 (Danso et al., 2018) and PET30 (Zhang et al., 2021) were shown here as controls.

In the following, a simple and fast protocol is described for the in vitro transcription and translation of metagenome-derived genes using an embodiment of the RNA polymerase of the invention, denoted RNAP_E in the following, to develop a cell-free screening pipeline for metagenome-derived resources. This is only an example of the multiple applications of the present invention. This example demonstrates the combined power of in silico sequence mining tools and CFPS systems for fast, efficient, and robust screening of metagenome-derived enzymes in high and semi-high-throughput screening platforms.

### MATERIALS and METHODS

### Bacterial strains, plasmids, and primers

Bacterial strains and plasmids as well as all primers used in this study are listed in Table 1 and Table 2, respectively. *Escherichia coli* (*E. coli*) strains for translation extract preparation and carrying gene clones were grown in LB medium (1 % tryptone/peptone, 0.5 % yeast extract, 0.5 % NaCl) supplemented with appropriate antibiotics (100 µg/mL ampicillin or 20 µg/mL kanamycin) at 37°C.

**Table 1. Bacterial strains, plasmids, and constructs used.**

| **Strains, plasmids and constructs** | **Traits** | **Reference/Source** |
|---|---|---|
| ***Strains*** | | |
| *E. coli* DH5α | F⁻ ϕ80 *lac*ZΔM15 Δ(*lacZYA-argF*)U169 *recAl endA1 hsdR*17(rki⁻, mk⁺)*phoA supE44* λ⁻ *thi*⁻1 *gyrA96 relAl* | Life Technologies (Frankfurt, Germany) |
| *E. coli* BL21(DE3) | F⁻ *ompT hsdS*_{B}(r_{B}⁻ m_{B}⁻) *gal dcm* (DE3) | Novagen/Merck (Darmstadt, Germany) |
| *E. coli* BL21-CodonPlus (DE3)-RIL | F⁻ *ompT hsdS*_{B}(r_{B}⁻ m_{B}⁻) *gal dcm* (DE3) *E. coli* B F⁻ *ompT hsdS*(r_{B}⁻ m_{B}⁻) dcm+ Tet^{r} *gal* λ(DE3) *endA* Hte [*argU ileY leuW* Cam^{r}] | Agilent Technologies (Waldbronn, Germany) |

| ***Vectors and Constructs*** | | |
|---|---|---|
| pET-21a(+) | 5.44 kb Expression vector, *lacI*, Amp^{R}, T7-promotor, C-terminal His₆-tag coding sequence | Novagen/Merck (Darmstadt, Germany) |
| pET-28a(+) | 5.37 kb Expression vector, *lacI,* Kan^{R}, T7-promotor, N- and C-terminal His₆-tag coding sequence | Novagen/Merck (Darmstadt, Germany) |
| pEX-A258::RNAP_E | Vector carrying synthesized gene coding for RNAP_E | This work |
| pET-28a(+)::RNAP _E | Expression vector carrying RNAP_E coding gene | This work |
| pET-21a(+)::sfGFP | Expression vector carrying sfGFP coding gene | This work |

**Table 2. List of primers used**

| **Primer Name** | **Sequence (5' - 3')** |
|---|---|
| T3_pET (Promoter a) | ATAAGATCTAATTAACCCTCACTAAAGGGGAATTGTGA GCGGATAAC (SEQ ID NO: 5) |
| SP6_pET (Promoter b) | ATAAGATCTATTTAGGTGACACTATAGGGGAATTGTGA GCGGATAAC (SEQ ID NO: 6) |
| T7_pET (Promoter c) | ATAAGATCTTAATACGACTCACTATAGGGGAATTGTGA GCGGATAAC (SEQ ID NO: 7) |
| EFN4HR8C_Upstr(12-30)_pET (Promoter d) | ATAAGATCTAAATACGCCAAGACTAGCGGGGAATTGT |
| | GAGCGGATAAC (SEQ ID NO: 8) |
| EFN4HR8C_Downstr(2 4-41)_pET (Promoter e) | ATAAGATCTTCAGAAGTCACACTATAAGGGAATTGTGA GCGGATAAC (SEQ ID NO: 9) |
| EFN4HR8C(rev)_3_pE T (Promoter f) | ATAAGATCTAATAGAAACATACTATAAGGGAATTGTGA GCGGATAAC (SEQ ID NO: 10) |
| EFN4HR8C(rev)_1_pE T (Promoter h) | ATAAGATCTAAATAAGACTCGCTAAGGGGAATTGTGA GCGGATAAC (SEQ ID NO: 11) |
| phiKDA1_Upstr(22-40)_pET (Promoter j) | ATAAGATCTGATTAACGTAGAGGATAGGGGAATTGTG AGCGGATAAC (SEQ ID NO: 12) |
| phiKDA1_5'(40-58)_pET (Promoter j) | ATAAGATCTCCTCAGCCATAACTAAGGGGGAATTGTGA GCGGATAAC (SEQ ID NO: 13) |
| phiKDA1(1231-48)_pET (Promoter k) | ATAAGATCTTACAACACTATACAATAGGGGAATTGTGA GCGGATAAC (SEQ ID NO: 14) |
| pET_rev | TCCGGATATAGTTCCTC (SEQ ID NO: 15) |

Underlined sequences are candidate promoters integrated into primers for template DNA preparation for transcription. All primers were synthesized at Eurofins MWG Operon (Ebersberg, Germany).

### Sequence-based mining of metagenome-based target genes

To construct a profile Hidden Markov Model (HMM) for database mining of RNA polymerase, sequences of known viral RNA-polymerases (T3, T7, SP6) were downloaded from NCBI (accession numbers: P07659 (v1), P00573 (v2), P06221 (v1)) and aligned using T-coffee 11.0.8 in accurate mode (Notredame et al., 2000). The resulting alignment was converted into a profile HMM using hmmbuild of the HMMER 3.1b2 package (Eddy, 2011). This model was subsequently used to screen assembled metagenomic datasets from IMG database. Only hits in complete open reading frames (ORFs) with a score >300 were retained. A viral RNA polymerase hit from the metagenome obtained from a feces sample of a six-year-old elephant was used for in vitro transcription of metagenome-derived genes (Ilmberger et al., 2014).

The same in silico mining strategy was applied for the prediction of poly(ethylene terephthalate) (PET) esterase candidates from metagenome databases (Pérez-García et al., 2021). 21 candidates after iterative sequence searches were selected and corresponding genes were synthesized into pET21a(+) vector after codon optimization (Biomatik, Wilmington, USA). PCR amplified DNA fragments with primers carrying promoter sequences (Table 2) were used as templates for CFPS and subsequent activity screening.

### Molecular cloning, expression and purification of recombinant RNAP_E

The gene sequence coding for the RNA polymerase (RNAP_E) from elephant metagenome is codon-optimized to the E.coli system and synthesized as pEX-A258::RNAP_E construct at Eurofins (Eurofins Genomics, Ebersberg, Germany). The gene was then re-cloned into pET-28a(+) expression vector by NheI and SalI restriction-modification. After verification via sequencing, the final construct was heat-shock transformed into a chemically competent E. coli BL21 (DE3) expression host. The His-tagged RNAP_E was induced at 28°C overnight with 0.3 mM isopropyl-b-D-thiogalactopyranoside. The purification was performed at 4 °C using Protino^{®} Ni-NTA Agarose (MACHEREY-NAGEL, Düren, Germany) according to the manufacturer's instruction in a lysis buffer (50 mM NaH2PO4 pH 8.0, 300 mM NaCl, 20 mM imidazole, 0.1 mM phenylmethylsulfonyl fluoride (PMSF), 1 mM dithiothreitol (DTT)). Then the agarose was washed with wash buffer (50 mM NaH2PO4 pH 8.0, 500 mM NaCl, 40 mM imidazole, 0.1 mM PMSF, 1 mM DTT). The protein was finally eluted with elution buffer (50 mM NaH2PO4 pH 8.0, 300 mM NaCl, 250 mM imidazole, 0.1 mM PMSF, 1 mM DTT). The eluted protein was dialyzed either in storage buffer (100 mM Tris-HCl pH 7.9, 100 mM KCl, 0.1 mM EDTA, 1 mM DTT, 0.1%Triton X-100, in diethylpyrocarbonate-treated H2O) or 2× storage buffer, which was then diluted with the same volume of glycerol. The purified protein was quantified using Bradford assay (Bradford, 1976) and stored either at 4 °C or -20 °C as indicated.

### RNAP_E promoter search and template DNA preparation

Promoter search was performed on the metagenomic contig which contains the coding sequence for RNAP E. The genome sequence of the best matching bacteriophage (phiKDA1) was also scanned for additional promoter candidates. After alignments and evaluation of candidate sequences using Geneious software version 10.1.2 (Biomatters, New Zealand), selected promoter sequences were integrated into primers (Table 2) to generate PCR-amplified DNA templates for transcription. Known bacteriophage promoter sequences of T7, T3, and SP6 (Table 2) were also included as controls.

### Preparation of translation extracts

A short and cost-effective protocol for translation extract preparation from strains of *E. coli* was pursued using the recommendations from Kwon and Jewett (2015). E. coli BL21-CodonPlus (DE3)-RIL (Agilent Technologies, Waldbronn, Germany) was grown in LB medium at 37°C to mid-exponential growth phase (OD600 ≈ 2.0). Then the cells were harvested by centrifugation at 5,000 × g for 15 min at 4°C. Cell pellets were washed three times with cold Buffer A (10 mM Tris-acetate pH 8.2, 14 mM magnesium acetate, 60 mM potassium glutamate, 2 mM DTT). Washed pellets were weighed, flash-frozen in liquid nitrogen and stored at -80°C for a maximum of three days before processing.

Cell pellets were thawed the next day and suspended in cold Buffer A (1 mL of Buffer A per 1 g of wet cell mass). Cells were lysed utilizing sonication (Output ctrl 0.5, duty cycle 50%) on ice. Cells were sonicated for 6 periods of 1 min each, with 1 min interval between each sonication. Then the cell debris and insoluble materials were removed by centrifugation at 4°C with a speed of 13,000 rpm twice, each time for 20 min. Finally, the cell extracts were aliquoted to 60 µL in RNase-free PCR tubes, flash-frozen in liquid nitrogen and immediately stored at - 80°C.

### In vitro transcription and in vitro translation

To synthesize the target protein in a cell-free system, in vitro transcription and translation reactions were performed in the un-coupled mode.

Independent from the translation reaction, in vitro transcription reaction was composed of 0.9 mM of each ribonucleoside triphosphates (rATP, rCTP, rGTP, rUTP, Promega, Mannheim, Germany), 20 mM of each essential amino acids, 0.1 U/µL of inorganic pyrophosphatase (NEB, Frankfurt am Main, Germany), 1 U/µL of RiboLock RNase inhibitor (Thermo Scientific, Darmstadt, Germany), 2 mM of spermidine, 10 mM of DTT, 6 mM MgCl2, 10 mM of NaCl mixed in 40 mM of Tris-HCl buffer (pH 7.8). A standard concentration of 1 µg PCR amplified linear DNA template was used in a 50 µL transcription reaction. The transcription mixture was incubated at 37°C for 2 hours followed by DNaseI (2 U/µg template DNA, Thermo Scientific) treatment for further 15 min at 37°C. Synthesized mRNA was then purified using RNA Clean & Concentrator^{™}-5 Kit from ZYMO Research (Freiburg, Germany). The quality and quantity of the mRNA were determined by NanoDrop 2000 Spectrophotometer (Thermo Scientific). The same reaction mixture was also used to characterize the activity of RNAP_E and RNAP_T7 (T7 RNA polymerase, Thermo Scientific) for evaluating promoter candidates. When temperature stability experiments of RNAPs were performed, the DNaseI treatment was skipped. At each time point mRNAs were immediately frozen at -80°C. The purified mRNAs were quantified with Qubit^{™} RNA HS Assay Kit in the Qubit^{®} 3.0 fluorometer (ThermoFisher Scientific, Darmstadt, Germany). The size of in vitro synthesized RNAs was checked with Agilent RNA 6000 Pico chip in the Agilent 2100 Bioanalyzer (Agilent Technologies) according to the manufacturer's instruction.

To synthesize the target protein, in vitro translation was performed with in vitro produced mRNA as template. A standard reaction mixture was composed of 1.2 U/µL of RiboLock RNase inhibitor, 0.35 mM of adenosine 5'-triphosphate (ATP), 0.33 mM of Nicotinamide adenine dinucleotide (NAD), 34 µg/mL folinic acid, 130 mM potassium glutamate, 10 mM ammonium acetate, 12 mM magnesium glutamate, 1.5 mM spermidine, 1 mM putrescine, 4 mM sodium oxalate and 0.27 mM coenzyme A (sodium salt hydrate), 23% (v/v) of translation extract with total volume of 60 µL. The reaction was incubated at 37°C for 4 hours followed by functional analysis.

### Functional enzyme assay with BHET

Most of PET esterases can break the ester bond of bis(2-hydroxyethyl) terephthalate (BHET) to produce terephthalate (TPA) and ethylene glycol (EG) with mono-(2-hydroxyethyl) terephthalate (MHET) as intermediate product (Danso et al., 2018;Joo et al., 2018;Pérez-García et al., 2021). Therefore, BHET is often selected as the representative substance to study the activity of PET esterase (Danso et al., 2018;Qiu et al., 2020). The yield of TPA and MHET can be quantified with UltiMate^{™} 3000 UHPLC system from Thermo Scientific with a Triart C18 column (YMC Europe GmbH, Dinslaken, Germany) and a VWD-3400 detector (Thermo Scientific) (Zhang et al., 2022). The data were analyzed with Compass HyStar software package from Bruker (Billerica, MA, USA).

### In vitro translation in polymersomes and flow cytometer analysis

Polymersome generation for in vitro protein synthesis based on double emulsion templates (Mastrobattista et al., 2005;Martino et al., 2012) and double emulsion generation by extrusion (Körfer et al., 2016) were reported previously. Briefly, double emulsion templates were generated by utilizing a mini extruder (Avanti, Polar Lipids, Inc., Alabaster, USA) by adding 200 µl of a mixture of chloroform and hexane at a volume ratio of 38:62, containing 1 mg/mL poly(butadiene)-b-poly(ethylene oxide) (PBD-b-PEO; MW: 1200 and 600, respectively) to 100 µl in vitro translation reaction mixture and pushing in total two times through a 10 µm PTFE membrane (Mitex PTFE hydrophobic membrane, Merck, Darmstadt, Germany) with the extruder. The (w/o) emulsion sample containing the in vitro translation reaction mixture and mRNA template of sfGFP was collected in 700 µl outer aqueous phase 0.1 M NaCl containing 10 wt% poly(vinyl alcohol) (PVA, MW 13000 - 23000, 87-89% hydrolyzed, Sigma-Aldrich^{®}) and was subsequently encapsulated in double emulsions by pushing it through a 12 µm Whatman membrane (Whatman Nuclepore Track-Etched Membranes, Sigma-Aldrich Biochemie GmbH, Hamburg, Germany) once. Polydisperse double emulsions encapsulating the in vitro translation reaction were transferred into a glass vial containing 0.15 M NaCl followed by polymersome formation and incubation at 37°C for 4 hours. After, in vitro translation of sfGFP in polymersomes were labeled with Nile Red and analyzed by fluorescence microscopy (BX51, Olympus, Tokyo, Japan). The emulsions were filtered through 50 µm filters (CellTrics, Sysmex Partec GmbH, Gorlitz, Germany) and analyzed with BD Influx cell sorter (Becton Dickinson Biosciences, Erembodegem, Belgium) as well as microscopy. Analysis was performed according to the forward scatter and to the sfGFP fluorescence intensity (λex 485 nm, λem 510 nm) after 488 nm excitation with a fluorescence emission detection of 530± 20nm (530/40(488)). The flow cytometer was operated at 5000 events-1 with a 100 µm nozzle and PBS (pH 7.4, 1.06 mM KH₂PO₄, 2.97 mM Na₂HPO₄, and 155.17 mM NaCl) as sheath fluid at.

### RESULTS

Since the well-known T7-RNA polymerase is a commercial product and has some draw backs with respect to moderate temperature stability and limitations during the transcription of linear fragments, we choose to identify a novel phage-derived RNA polymerase for the setup of our pipeline. Therefore, in this study, we have first characterized a novel RNA polymerase, designated RNAP_E, which can be applied for the in vitro metagenomic screening.

### Identification of RNAP_E from the elephant feces microbiota

A total of 107 full-length viral RNA polymerase hits with more than a score of 300 were selected from 30 metagenome datasets from IMG database using HMM derived from T7, T3, and SP6 RNA polymerase sequences. About 131x10E5 gene counts were screened from a total of 66 Gb assembled DNA of the metagenome datasets. A single full-length RNA polymerase hit, which is encoded on an environmental phage contig, was obtained from elephant feces (Figure 1A) (Ilmberger et al., 2014). The phylogenetic assignment implied that it most likely belongs to the genus of the *Drulisvirus* within the family of *Autographiviridae* and the Phylum of the *Uroviricota.* It is located on a 10 kbp scaffold with a DNA polymerase, two endonucleases and many hypothetical proteins (Figure 1A). The RNAP_E gene was predicted to encode for an 816-amino-acid protein. The predicted amino acid sequence carries the COG5108 domain, with homologies to mitochondrial DNA-directed RNA polymerases, Pfam 00940 RNA_pol and the Pfam RPOLN signatures and it belongs to EC:2.7.7.6 - DNA-directed RNA polymerase. While the RNAP_E encoding contig does not encode the complete phage, a more detailed data analysis showed that RNAP_E is similar to phages found in *Klebsiella* and other *Enterobacteria* with the highest homologies (71%) observed to a DNA-directed RNA polymerase from *Enterobacter cloacae* phage phiKDA (NCBI Reference Sequence YP_009167685.1).

After codon optimization and overexpression in *E. coli* BL21 (DE3), up to 18 mg of the recombinant His-tagged RNAP_E protein could be purified from a liter of expression culture (Figure 1B). RNAP_E is a single subunit RNA polymerase and has only 26% identity (calculated by NCBI-Blast-Global Alignment) with T7 RNA polymerase based on amino acid alignment. Compared to other known bacteriophage RNA polymerases, RNAP_E is phylogenetically located in a separate branch (Figure 1C), representing that it might carry a different evolutionary process from the others. The amino acid sequence alignment showed that the most conserved domain is the palm-subunit domain (according to the RNAP_T7 protein sequence, (Borkotoky and Murali, 2018), Figure 2), which is important for the catalytic activity. However, the finger sub-domains, which are critical for the template-binding, exhibit much lower similarity among these RNA polymerases (Figure 2). Comparison of predicted RNAP_E with the crystal structure of RNAP_T7 (PDB ID: 1CEZ, (Cheetham et al., 1999)) clearly showed that the promoter-binding regions (including the finger sub-domain) of these two proteins fold quite differently, indicating that they might recognize different promoter sequences.

### Promoter search for RNAP_E

HMM logo from the alignment of candidate promoters are presented in Figure 3A. Regions of promoter binding were given special attention while choosing candidate promoter sequences. Of all tested promoters, RNAP_E was most active with a promoter sequence 5'-TCAGAAGTCACACTATAA-3' and designated as Promoter e (Figure 3B; SEQ ID NO: 2). This promoter sequence is located 24 bp upstream of the RNAP_E coding region and has 67% identity (calculated by NCBI-Blast-Global Alignment) with the T7 promoter sequence. Interestingly, the RNAP_T7 could not initiate transcription from this promoter (Figure 3B). RNAP_E was also found to be moderately active on other candidate promoter sequences 5'-ATTTAGGTGACACTATAG-3' (SP6 promoter, indicated as Promoter b; SEQ ID NO: 4) and 5'-AATAGAAACATACTATAA-3' (SEQ ID NO: 3; denoted Promoter f, also a consensus promoter sequence on the scaffold, where RNAP_E was found), which have 61% and 68% sequence identity (calculated by NCBI-Blast-Global Alignment) with the Promoter e, respectively (Figure 3B). All of the other candidate promoter sequences including the ones from bacteriophage phiKDA1 (Promoter i-k) did not show any transcription activity with the RNAP_E. RNAP_E did not show any sign of activity on T3 and T7 promoter sequences, either (Promoter a and c, Figure 3B). Moreover, RNAP_E can't recognize the promoters for the other single subunit RNA polymerases from marine cyanophage Syn5 and Klebsiella phage KP34 (Data not shown). Therefore, Promoter e is a specific promoter for RNAP_E. The initial characterizations of RNAP__E and transcription of enzyme-coding genes were done employing DNA templates carrying Promoter e.

### Characterization of the activity of RNAP_E in in vitro transcription

To test the efficiency of RNAP__E for in vitro transcription, 0.1 - 25 µg of RNAP__E was added in the 50 µL of transcription reaction using 1 µg of DNA template - PET2 DNA sequence with Promoter e (PET2 is a well-characterized PET esterases (Danso et al., 2018)). The results revealed that the highest yield of mRNA was produced by 2.5 µg or 5 µg of RNAP_E. The produced mRNA level is at the comparable range with the mRNA in vitro produced by 56 units of RNAP_T7, which is suggested by the manufacturer. When the amount of RNAP_E was less than 2.5 µg per reaction, not all the templates could be in vitro transcribed; when more than 5 µg of RNAP_E was added, the yield of mRNA decreased too, it might be because of the competing binding between the template and the RNA polymerase. Therefore, 5 µg of RNAP_E was generally used per reaction in a 50 µL transcription reaction mix with 1 µg of DNA as template. Normally, between 50 - 90 mg mRNA could be generated from one such reaction.

To find out the best long-term storage condition for RNAP_E, the purified protein was stored at two different temperatures (4°C and -20°C) in the buffer either with or without 50% glycerol. Its activity was tested by measuring the amount of in vitro produced mRNA. The results showed that RNAP_E is still fully active for up to 50 days in all tested storage conditions and glycerol did not influence the activity of RNAP_E. However, after a few days, there were some visible precipitates in the sample stored only in buffer without glycerol, indicating that glycerol might increase the solubility of RNAP_E. Therefore, RNAP_E was routinely stored in the storage buffer with 50% glycerol at -20°C. The RNAP_E in a buffer with 50% glycerol was still highly active after one-year storage at -20°C (data not shown).

The activity of RNAP_E was further tested at different temperatures. As indicated in Figure 4A, RNAP_E exhibits the highest activity at 37°C. RNA was continually produced at 37°C and 28°C and the maximal production was achieved after 1 to 2 hours of incubation. RNAP_E was also active at 45°C, 55°C, 65°C, and 75°C. However, at these elevated temperatures the initial activity declined rapidly and the enzymes were not active after one hour. Nevertheless, compared to the activity at 37°C after one hour, RNAP E still exhibits around 52% activity at 45°C and 55°C and 29-31% activity at 65°C and 75°C (Figure 4A).

The velocity of the mRNA synthesis at 37°C and 55°C with RNAP_E was assayed in comparison to the well-known RNAP_T7 (Figure 4B). Generally, the quality of produced RNAs by RNAP_E and RNAP_T7 appeared to be similar. Both can produce high purity of RNAs with the size of around 1140 nucleotides (nt) and using the PET2 coding gene as a template. Interestingly, RNAP_E has a greater velocity at both temperatures compared to RNAP_T7 (Figure 4B). According to the absolute amount of mRNA produced over 0-25 minutes, RNAP_E was 3.2-fold faster at 37°C than RNAP_T7. Even at 55°C RNAP_E was still 1.7-fold faster compared to RNAP_T7. Therefore, within a short period, RNAP_E exhibits higher efficiency for in vitro transcription at these elevated temperatures.

### Application of RNAP_E in metagenomics screening with in vitro expression system

Metagenomics screening is a very useful tool for searching a new functional enzymes. While it gives fast access to genes of interests, the predicted candidate genes need to be verified with respect to their activities. Cloning and expressing each gene heterologously is time consuming and tedious if a large number of clones need to be analyzed. Therefore, a cell-free metagenomics screening protocol was developed with the above RNAP_E, and using PET esterase genes as examples.

For demonstration reasons, an example of a sequence mining process reported for the prediction of PET esterases (Danso et al., 2018; Pérez-García et al., 2021) was applied here. PET esterases genes were chosen because they are difficult to be found and only less than 30 such enzymes have been published (Zhang et al., 2022). Accordingly, 21 predicted PET-active candidate genes, together with well-characterized PET2 (Danso et al., 2018) and PET30 (Zhang et al., 2022), were synthesized after codon optimization for E. coli system. Following the gene synthesis a single PCR reaction with 30 cycles was performed to introduce a specific promoter for RNAP_E (designated Promoter e) upstream (5'-prime) of the target genes (see Materials and Methods, and (Han et al., to be published)). PCR products were purified and transcribed into mRNA with RNAP_E (as outlined in Materials and Methods). All 21 candidate genes and the two controls PET2 and PET30 were successfully transcribed in vitro by RNAP_E (Figure 5A). All RNAs exhibited the correct size on a RNA gel annotated using Bioanalyzer and Agilent 2100 Expert software (Figure 5A).

Following the mRNA synthesis and quality control, the mRNAs were translated using ribosomal complexes from E. coli BL21-CodonPlus (DE3)-RIL cell extracts. After translation with in vitro produced mRNA as template in the self-established cell-free system, all produced candidates were applied to an activity assay using BHET as substrate. Notably from the 23 translation assays, 14 positive samples including the two positive controls were observed. Thereby the amount of produced TPA showed that these candidates exhibited different enzymatic activity concerning the degradation of BHET at the tested temperatures (Figure 5B). PET2 is active at both 28°C and 55°C, while PET30 is only active at a lower temperature, which is consistent with the previous work (Danso et al., 2018; Nakamura et al., 2021; Zhang et al., 2022). According to the yield of TPA, C20 shows the highest activity among screened 21 candidates on the degradation of BHET at 55°C and it is also quite active at 28°C, but much lower than 55°C; candidate C15 exhibits highest activity at 28°C and mild activity at 55°C. Candidate C9 - C13, and C17 are also active at 28°C. Besides, C2, C8, C18, and C19 did release a significant amount of MHET instead of TPA at 28°C, adding another 4 active enzymes (Figure 5C). Moreover, C9 - C12 produced significantly higher amount of MHET than the negative control, indicating that they are also active at 55°C (Figure 5C). Thus, using this in vitro metagenomics screening method and employing RNAP_E 12 novel enzyme candidates have been identified. These candidate genes have not been identified as BHET-active enzymes in previous work.

Further, it was investigated, if other genes could be expressed based on an in vitro pipeline using RNAP_E. Therefore the metagenome-derived cellulase CelA2 (Ilmberger et al., 2012), the lipase CalB (Uppenberg et al., 1994), and the superfolder green fluorescent protein (sfGFP, (Pédelacq et al., 2006)) were expressed using the cell-free protein expression system. In all cases, the amount of produced proteins were sufficient to perform the initial activity assay. In the case of PET esterase and cellulase, the presence of translation mix or reaction components in the assay did not have significant impact on the yield. Therefore, the translation mix was directly applied to the activity assays. In the case of the expressed lipase, the in vitro expressed lipase was first immobilized to a 96-well nickel-coated microtiter plate to select only His-tagged lipase out of the translation extract, thereby removing the background lipolytic activities from the extract. Furthermore, sfGFP has been expressed with RNAP_E in cell-free polymersomes instead of normal reaction tubes. Such polymersomes with sfGFP enable the development of next-generation ultrahigh-throughput functional screening of metagenomes based on flow cytometry (Markel et al., 2020). All the above examples suggested that in vitro expressed proteins are active, and they exhibit detectable activity. Moreover, in vitro expression system is a suitable tool to express a small number of proteins (20-50) in short time periods.

### References

Borkotoky, S., and Murali, A. (2018). The highly efficient T7 RNA polymerase: A wonder macromolecule in biological realm. International Journal of Biological Macromolecules 118, 49-56.

Bradford, M.M. (1976). A rapid and sensitive method for the quantitation of microgram quantities of protein utilizing the principle of protein-dye binding. Analytical Biochemistry 72, 248-254.

Büscher, N., Sayoga, G.V., Rübsam, K., Jakob, F., Schwaneberg, U., Kara, S., and Liese, A. (2019). Biocatalyst immobilization by anchor peptides on an additively manufacturable material. Organic Process Research & Development 23, 1852-1859.

Cheetham, G.M.T., Jeruzalmi, D., and Steitz, T.A. (1999). Structural basis for initiation of transcription from an RNA polymerase-promoter complex. Nature 399, 80-83.

Danso, D., Schmeisser, C., Chow, J., Zimmermann, W., Wei, R., Leggewie, C., Li, X., Hazen, T., and Streit, W.R. (2018). New insights into the function and global distribution of polyethylene terephthalate (PET)-degrading bacteria and enzymes in marine and terrestrial metagenomes. Applied and Environmental Microbiology 84, e02773-02717.

Eddy, S.R. (2011). Accelerated Profile HMM Searches. PLoS Comput Biol 7, e1002195.

Ekkers, D.M., Cretoiu, M.S., Kielak, A.M., and Elsas, J.D. (2012). The great screen anomaly--a new frontier in product discovery through functional metagenomics. Appl Microbiol Biotechnol 93, 1005-1020.

Ferrer, M., Martinez-Martinez, M., Bargiela, R., Streit, W.R., Golyshina, O.V., and Golyshin, P.N. (2016). Estimating the success of enzyme bioprospecting through metagenomics: current status and future trends. Microbial Biotechnology 9, 22-34.

Gabor, E.M., Alkema, W.B., and Janssen, D.B. (2004). Quantifying the accessibility of the metagenome by random expression cloning techniques. Environmental microbiology 6, 879-886.

Grimm, A.R., Sauer, D.F., Mirzaei Garakani, T., Rübsam, K., Polen, T., Davari, M.D., Jakob, F., Schiffels, J., Okuda, J., and Schwaneberg, U. (2019). Anchor peptide-mediated surface immobilization of a Grubbs-Hoveyda-type catalyst for ring-opening metathesis polymerization. Bioconjugate Chemistry 30, 714-720.

Han, Y., Dierkes, R.F., and Streit, W.R. (to be published). "Metagenomic screening of a novel PET esterase via in vitro expression system," in Metagenomics: Methods and Protocols, eds. W.R. Streit & R. Daniel. (New York, NY: Springer New York).

Ilmberger, N., Güllert, S., Dannenberg, J., Rabausch, U., Torres, J., Wemheuer, B., Alawi, M., Poehlein, A., Chow, J., Turaev, D., Rattei, T., Schmeisser, C., Salomon, J., Olsen, P.B., Daniel, R., Grundhoff, A., Borchert, M.S., and Streit, W.R. (2014). A comparative metagenome survey of the fecal microbiota of a breast- and a plant-fed Asian elephant reveals an unexpectedly high diversity of glycoside hydrolase family enzymes. PLoS One 9, e106707.

Ilmberger, N., Meske, D., Juergensen, J., Schulte, M., Barthen, P., Rabausch, U., Angelov, A., Mientus, M., Liebl, W., Schmitz, R.A., and Streit, W.R. (2012). Metagenomic cellulases highly tolerant towards the presence of ionic liquids-linking thermostability and halotolerance. Applied Microbiology and Biotechnology 95, 135-146.

Joo, S., Cho, I.J., Seo, H., Son, H.F., Sagong, H.-Y., Shin, T.J., Choi, S.Y., Lee, S.Y., and Kim, K.-J. (2018). Structural insight into molecular mechanism of poly(ethylene terephthalate) degradation. Nature Communications 9, 382.

Jorgensen, E.D., Durbin, R.K., Risman, S.S., and Mcallister, W.T. (1991). Specific contacts between the bacteriophage T3, T7, and SP6 RNA polymerases and their promoters. Journal of Biological Chemistry 266, 645-651.

Kinfu, B.M., Jahnke, M., Janus, M., Besirlioglu, V., Roggenbuck, M., Meurer, R., Vojcic, L., Borchert, M., Schwaneberg, U., Chow, J., and Streit, W.R. (2017). Recombinant RNA polymerase from Geobacillus sp. GHH01 as tool for rapid generation of metagenomic RNAs using in vitro technologies. Biotechnology and Bioengineering 114, 2739-2752.

Körfer, G., Pitzler, C., Vojcic, L., Martinez, R., and Schwaneberg, U. (2016). In vitro flow cytometry-based screening platform for cellulase engineering. Scientific Reports 6, 26128. Kwon, Y.-C., and Jewett, M.C. (2015). High-throughput preparation methods of crude extract for robust cell-free protein synthesis. Scientific Reports 5, 8663.

Lam, K.N., Cheng, J., Engel, K., Neufeld, J.D., and Charles, T.C. (2015). Current and future resources for functional metagenomics. Frontiers in Microbiology 6.

Lehmann, C., Sibilla, F., Maugeri, Z., Streit, W.R., De Maria, P.D., Martinez, R., and Schwaneberg, U. (2012). Reengineering CelA2 cellulase for hydrolysis in aqueous solutions of deep eutectic solvents and concentrated seawater. Green Chemistry 14, 2719-2726.

Markel, U., Essani, K.D., Besirlioglu, V., Schiffels, J., Streit, W.R., and Schwaneberg, U. (2020). Advances in ultrahigh-throughput screening for directed enzyme evolution. Chemical Society Reviews 49, 233-262.

Martino, C., Kim, S.H., Horsfall, L., Abbaspourrad, A., Rosser, S.J., Cooper, J., and Weitz, D.A. (2012). Protein expression, aggregation, and triggered release from polymersomes as artificial cell-like structures. Angewandte Chemie (International ed. in English) 51, 6416-6420. Mastrobattista, E., Taly, V., Chanudet, E., Treacy, P., Kelly, B.T., and Griffiths, A.D. (2005). High-throughput screening of enzyme libraries: in vitro evolution of a beta-galactosidase by fluorescence-activated sorting of double emulsions. Chemistry & Biology 12, 1291-1300. Nakamura, A., Kobayashi, N., Koga, N., and Iino, R. (2021). Positive charge introduction on the surface of thermostabilized PET hydrolase facilitates PET binding and degradation. ACS Catalysis 11, 8550-8564.

Notredame, C., Higgins, D.G., and Heringa, J. (2000). T-coffee: a novel method for fast and accurate multiple sequence alignment11Edited by J. Thornton. Journal of Molecular Biology 302, 205-217.

Pédelacq, J.D., Cabantous, S., Tran, T., Terwilliger, T.C., and Waldo, G.S. (2006). Engineering and characterization of a superfolder green fluorescent protein. Nature Biotechnology 24, 79-88.

Pérez-García, P., Danso, D., Zhang, H., Chow, J., and Streit, W.R. (2021). "Chapter Seven - Exploring the global metagenome for plastic-degrading enzymes," in Methods in Enzymology, eds. G. Weber, U.T. Bornscheuer & R. Wei. Academic Press), 137-157.

Perner, M., Ilmberger, N., Kohler, H.U., Chow, J., and Streit, W.R. (2011). "Emerging Fields in Functional Metagenomics and Its Industrial Relevance: Overcoming Limitations and Redirecting the Search for Novel Biocatalysts," in Handbook of Molecular Microbial Ecology II. John Wiley & Sons, Inc.), 481-498.

Qiu, L., Yin, X., Liu, T., Zhang, H., Chen, G., and Wu, S. (2020). Biodegradation of bis(2-hydroxyethyl) terephthalate by a newly isolated Enterobacter sp. HY1 and characterization of its esterase properties. Journal of Basic Microbiology 60, 699-711.

Rübsam, K., Stomps, B., Böker, A., Jakob, F., and Schwaneberg, U. (2017). Anchor peptides: A green and versatile method for polypropylene functionalization. Polymer 116, 124-132. Steele, H.L., Jaeger, K.E., Daniel, R., and Streit, W.R. (2009). Advances in recovery of novel biocatalysts from metagenomes. J Mol Microbiol Biotechnol 16, 25-37.

Streit, W.R., and Schmitz, R.A. (2004). Metagenomics - the key to the uncultured microbes. Current Opinion in Microbiology 7, 492-498.

Tamura, K., Stecher, G., Peterson, D., Filipski, A., and Kumar, S. (2013). MEGA6: molecular evolutionary genetics analysis version 6.0. Molecular Biology and Evolution 30, 2725-2729. Uchiyama, T., and Miyazaki, K. (2009). Functional metagenomics for enzyme discovery: challenges to efficient screening. Current Opinion in Biotechnology 20, 616-622.

Uppenberg, J., Hansen, M.T., Patkar, S., and Jones, T.A. (1994). The sequence, crystal structure determination and refinement of two crystal forms of lipase B from Candida antarctica. Structure 2, 293-308.

Zhang, H., Perez-Garcia, P., Dierkes, R.F., Applegate, V., Schumacher, J., Chibani, C.M., Sternagel, S., Preuss, L., Weigert, S., Schmeisser, C., Danso, D., Pleiss, J., Almeida, A., Höcker, B., Hallam, S.J., Schmitz, R.A., Smits, S.H.J., Chow, J., and Streit, W.R. (2022). The Bacteroidetes Aequorivita sp. and Kaistella jeonii produce promiscuous esterases with PET-hydrolyzing activity. Frontiers in Microbiology 12

## Claims

1. A single-subunit RNA polymerase, ssRNAP, the ssRNAP comprising
a) an amino acid sequence according to the sequence of SEQ ID NO: 1, or
b) an amino acid sequence having at least 75 % sequence identity with the amino acid sequence of SEQ ID NO: 1.

2. An ssRNAP according to claim 1, wherein the ssRNAP has at least 80 %, preferably at least 85 %, 90%, 95%, 96%, 97%, 98%, 99%, or at least 99.5% sequence identity with the amino acid sequence of SEQ ID NO: 1.

3. A fragment of an ssRNAP according to one of claims 1 or 2, wherein the fragment comprises at least 60, preferably at least 65, 70, 75, 80, 85, 90, 100, 110 or at least 120 consecutive amino acids of the sequence of SEQ ID NO: 1, further preferred at least 150, 175, 200, 250, 300, 350, 400, 450, 500, 550, 600, 650, 700 or at least 750 consecutive amino acids of said ssRNAP, and wherein the fragment has RNA polymerase activity.

4. A fusion protein, comprising an ssRNAP according to one of claims 1 or 2, or a fragment according to claim 3, being fused to a protein having an enzymatic activity different from an RNA polymerase.

5. The fusion protein according to claim 4, wherein the protein has mRNA capping activity.

6. A nucleic acid being or comprising a nucleic acid encoding an ssRNAP according to one of claims 1 or 2, a fragment according to claim 3, or a fusion protein according to claims 4 or 5.

7. A nucleic acid comprising a promoter and a transcription unit functionally linked to the promoter, the promoter being or comprising
a) the nucleotide sequence of SEQ ID NO: 2 or 3, or
b) a nucleotide sequence having at least 75 % sequence identity with the nucleotide sequence of SEQ ID NO: 2 or 3, the nucleotide sequence having promoter activity in relation to the ssRNAP according to one of claims 1 or 2.

8. The nucleic acid according to claim 7, the promoter being or comprising a nucleotide sequence having at least 80 %, preferably at least 85 %, 90%, 95%, 96%, 97%, 98%, 99%, or at least 99.5% sequence identity with the nucleotide sequence of SEQ ID NO: 2 or 3, preferably SEQ ID NO: 2, the nucleotide sequence having promoter activity in relation to the ssRNAP according to one of claims 1 or 2, or the fragment according to claim 3, and/or the fusion protein according to one of claims 4 or 5.

9. A vector comprising a nucleic acid according to one of claims 6, 7 or 8.

10. A host cell being transformed with a nucleic acid according to one of claims 6, 7 or 8, or with a vector according to claim 9.

11. Use of an ssRNAP according to one of claims 1 or 2, or a fragment thereof according to claim 3, or a fusion protein according to claim 4 or 5, for the biotechnological production of RNA, preferably mRNA, or a RNA/DNA hybrid molecule.

12. A method of amplifying a target nucleic acid in vitro, comprising contacting an ssRNAP according to one of claims 1 or 2, or a fragment thereof according to claim 3, or a fusion protein according to one of claims 4 or 5, with a DNA template comprising the target nucleic acid, and with nucleoside triphosphate molecules suitable or necessary for the in vitro transcription of the target nucleic acid, under conditions suitable for a transcription of the target nucleic acid to occur.

13. The method of claim 12, wherein the target nucleic acid is functionally linked to a promoter having the sequence according to SEQ ID NO: 2 or 3, or to a promoter having a nucleotide sequence having at least 75 % sequence identity, preferably at least 80 %, 85 %, 90%, 95%, 96%, 97%, 98%, 99%, or at least 99.5% sequence identity with the nucleotide sequence of SEQ ID NO: 2 or 3, the nucleotide sequence having promoter activity in relation to the ssRNAP according to one of claims 1 or 2, or the fragment according to claim 3, and/or the fusion protein according to one of claims 4 or 5.

14. The method of one of claims 12 or 13, wherein the target nucleic acid is an mRNA therapeutic, preferably an mRNA vaccine or an mRNA for use in gene therapy.

15. A kit for carrying out the method according to one of claims 12 to 14, comprising an ssRNAP according to one of claims 1 or 2, or a fragment thereof according to claim 3, or a fusion protein according to claim 4 or 5, and, optionally, a buffer suitable for the transcription of the target nucleic acid to occur.
